Europäisches Patentamt

European Patent Office

Office européen des brevets

(11) Publication number: **0 430 388 A2**

# EUROPEAN PATENT APPLICATION

(21) Application number: 90305063.1

(22) Date of filing: **10.05.90**

(51) Int. Cl.⁵: **A61K 31/135**

The title of the invention has been amended (Guidelines for Examination in the EPO, A-III, 7.3).

(30) Priority: **20.11.89 GB 8926171**

(43) Date of publication of application:
**05.06.91 Bulletin 91/23**

(84) Designated Contracting States:
**AT BE CH DE DK ES FR GB GR IT LI LU NL SE Bulletin**

(71) Applicant: **Applied Research Systems ARS Holding N.V.**
**6 John B. Gorsiraweg**
**Curaçao(AN)**

(72) Inventor: **Baird, David T. Dept. of Obstetr. & Gynaecol.**
**University of Edinburgh Centr. for Reprod. Biol.**
**37, Chalmers Street Edinburgh EH3 9EW(GB)**
Inventor: **Glasier, Anna F**
**Dept.of Obst.& Gyn. Univ.of Edin.Cent.for Rep.Biol**
**37,Chalmers Street Edinburgh EH 3 9EW(GB)**

(74) Representative: **Hildyard, Edward Martin et al**
**Frank B. Dehn & Co. European Patent Attorneys Imperial House 15-19 Kingsway London WC2B 6UZ(GB)**

(54) **Use of clomiphene for treatment of infertility.**

(57) (2-{para-(2-Chloro-1,2-diphenylvinyl) phenoxy}triethylamine or a bioequivalent derivative thereof from which the Zu isomer is substantially absent, may be advantageously used for the treatment of female infertility by the administration of a pharmacologically effective amount thereof.

EP 0 430 388 A2

## TREATMENT OF INFERTILITY

This invention concerns the treatment of female infertility by use of the known triphenylethylene compound (2-{para(2-chloro-1,2-diphenylvinyl) phenoxy}triethylamine (hereinafter referred to under its common name clomiphene or clomid). The term "clomiphene" is used herein generically to denote both the free base and bioequivalent derivatives thereof, especially acid addition salts containing pharmacologically acceptable anions. The citrate is the preferred acid addition salt.

Clomiphene is a synthetic oestrogen agonist-antagonist which has been widely used for the induction of ovulation in anovulatory women for many years (Greenblatt et al (1961); J. Am. Med. Ass. 178:101-104)

Most investigators believe that clomiphene acts by inhibiting the negative effect of endogenous oestrogens on the hypothalamus. This inhibition results in the elevation of gonadotropins which in turn causes follicular growth and ovulation. Therefore, the hormonal action of clomiphene is generally considered to be antioestrogenic in the human. However, clear-cut evidence exists, both in animal and human studies, that this drug is also oestrogenic. These conflicting and sometimes paradoxical results have lead to considerable confusion concerning the mechanism of action of clomiphene.

Clomiphene is a racemic mixture of cis and trans isomers. The original cis and trans isomers were called isomer-B and isomer-A respectively. In 1976, it was revealed that the cis and trans nomenclature had been mistakenly reversed (Ernst et al. (1976); J. Pharm. Sci 65 : 148-150). It is important to keep these changes in mind when comparing the literature published before and after 1976 (see Table 1 for changes and current status).

TABLE 1.

| Changes in the Nomenclature of Clomiphene | | |
|---|---|---|
| Original Name | Prior to 1976 | After 1976 |
| Isomer A | Trans-clomiphene | Cis-clomiphene or Zuclomiphene |
| Isomer B | Cis-clomiphene | Trans-clomiphene or Enclomiphene |

Currently the authentic cis and trans isomers are called Zuclomiphene (with oestrogenic activity) and Enclomiphene (with antioestrogenic activity) respectively.

The racemic mixture, which is administered to induce ovulation in women, is generally 38% zuclomiphene (cis) and 62% Enclomiphene (trans).

A better understanding of the complex effects of clomiphene in vivo is hampered by the fact that the commercially available preparations used in clinical practice are mixtures of the two isomeric forms. Each of these isomers has the capacity to interact with oestrogen receptors and exhibit oestrogenic or anti-oestrogenic effects depending on the kind of tissue and the species, as well as the dose. J. H. Olsson et al., (Human Reproduction 2 (6), 463-468, 1987) concluded that Enclomiphene and Zuclomiphene have similar inhibitory effects on progestin synthesis in cultured human granulosa cells in vitro.

In spite of the widespread use of clomiphene for over 20 years in the treatment of female infertility, the results of such therapy have not been uniformly successful. In particular, it has been observed that during clomiphene therapy in infertile women, the pregnancy rate (30-40%) is substantially lower than the ovulation rate (70-90%) (Wu et al.: Clin. Obstet.

Gynecol. 27, 953-965, 1984). An association between clomiphene therapy and luteal phase deficiency has also been reported (Coek et al.: Ann. J. Obstet. Gynecol. 149, 613-616, 1984).

It has been proposed that these "side effects" of clomiphene are due to inadequate gonadotrophic stimulation during the follicular phase (Garcia et al: Fertil. Steril. 28, 707-717, 1977), but also that clomiphene has a negative direct effect on the ovary (Adashi: Fertil. Steril. 41, 331-344, 1984).

According to Mikkelson et al. (Fertility and Sterility, 46 (3) 392, 1986), the Zu (cis) isomer is the active one.

On the contrary, Glasier et al: Human Reproduction 4 (33), 252-256, 1989, conclude that the En isomer is the isomer active in inducing follicular development. However, the spinnbarkeit of the mucus was significantly reduced in Zu + En (racemate) treated cycles and En isomer treated cycles, but there was no reduction in Zu isomer treated cycles.

This detrimental effect of the racemate and of the En isomer on the uterus cervix and its mucus is claimed to be one of the factors responsible for the discrepancy between ovulation rate and pregnancy rate (Fertil. Steril. 37, 161-167, 1982; Obstet. Gynaecol. 41, 602-607, 1973; obstet. Gynaecol. 39, 389, 1972).

There is therefore a need to arrive at improved dosing regimens which reduce the negative side effects associated with the administration of the clomiphene preparations which are currently commercially available.

We have now surprisingly found that the elimination of the Zu isomer from the racemic mixture, i.e. the administration of a pharmacologically active amount of En isomer, does not exert any adverse influence on the uterus cervix and its mucus. The ovulation rate is not affected either. Moreover, the potential teratogenic effects of racemic clomiphene are reduced if not eliminated.

One aspect of our invention is, therefore, a method for the treatment of female infertility which comprises administering, preferably in the early follicular phase of the menstrual cycle, a pharmacologically effective amount of clomiphene (as defined herein) from which the Zu (cis) isomer is substantially absent.

Another aspect of our invention is the use of clomiphene (as defined herein) from which the Zu (cis) isomer is substantially absent, to prepare a pharmaceutical composition for the treatment of female infertility.

The use of clomiphene substantially free of the Zu isomer, i.e. the use of the En isomer substantially free of the Zu isomer, according to the present invention provides effective treatment of female infertility at lower dosages than have been used in conventional clomiphene therapy. This is desirable by reason of the suspected toxicity of clomiphene to oocytes and embryos (Schmidt et al, Am. J. Obstet. Gynecol, 154 (4), 727). Although clomiphene is generally regarded as a safe and clinically-proven drug, significant toxic and teratogenic effects have been demonstrated on animals (reviewed by Clark and Markaverich, Pharmac. Ther. 15 : 467-569 at 500-506, 1982). Thus a reduction in dosage without loss of clinical effectiveness is a major advance in the use of clomiphene according to the present invention.

Typical dosages of clomiphene free of the Zu isomer suggested for treatment of the infertile human female are e.g. about 10-75 mg per diem, preferably 20-50 mg per diem. Racemic clomiphene is generally used at about 100 mg per diem. All doses are calculated as the citrate.

The preferred administration protocol according to the present invention involves administration of the active component in the early part of the menstrual cycle, generally over a time period in the first third of the cycle. A typical protocol would involve administration on days 5 to 9 of the oestrus cycle. An alternative, new, preferred protocol according to the present invention involves administration on days 3 to 7 of the menstrual cycle. Preferred patients are anovlatory, and free from any residual effects of prior treatment with clomiphene.

The clomiphene free of the Zu isomer will generally be administered per os e.g. as the citrate, since it is well absorbed by the oral route and this mode of administration is the most convenient for the patient.

A method to separate the En and Zu isomers of clomiphene is well known in the art (see DE 2,212,660).

Suitable forms for administration include tablets, capsules, syrups, emulsions and dispersible powders. Suitable tablets may be prepared, for example, by mixing the active substance with known adjuvants, for example inert diluents such as calcium carbonate, calcium phosphate or lactose, disintegrants such as corn starch or alginic acid, binders such as starch or gelatine, lubricants such as magnesium stearate or talc and/or agents for obtaining delayed release such as carboxypolymethylene, carboxymethyl-cellulose, cellulose acetate phthalate or polyvinyl acetate. The tablets may also consist of several layers.

Coated tables may be prepared in the same way by coating cores produced analogously to the tablets with substances conventionally used for tablet coating, e.g. collidone or shellac, gum arabic, talc, titanium dioxide or sugar. In order to obtain delayed release, the core may also consist of several layers. Similarly, the tablet coating may consist of several layers in order to achieve delayed release, using the excipients given above for the tablets.

Syrups of the active substance may additionally contain a sweetener such as saccharin, cyclamate, glycerine or sugar and a flavour-enhancing agent, e.g. a flavouring such as vanillin or orange extract. They may also contain suspension adjuvants or thickeners such as sodium carboxymethylcellulose, wetting agents, e.g. condensation products of fatty alcohols with ethylene oxide, or preservatives such as p-hydroxybenzoates.

Capsules containing the active substance may be prepared, for example, by mixing the active substances with inert vehicles such as lactose or sorbitol and encapsulating them in gelatine capsules.

It is, of course, also possible to administer the Enclomiphene by other routes, e.g. parenteral or rectal Formulations for such use may be prepared by conventional procedures of pharmacy.

EXAMPLE

Normoprolactinaemic women with regular menstrual cycles and patent Fallopian tubes, mean age 32 years, were monitored over three menstrual cycles. The patients were treated with 100 mg of clomiphene citrate daily from day 2 of the first cycle for 5 days. Following a "washout" control cycle with no treatment, the patients were then selected randomly into two groups to receive 50 mg daily of either the En isosmer (9 patients) or the Zu isomer (10 patients) from day 2 of the third cycle for 5 days.

Daily samples of urine were analysed for oestrone, pregnanediol, LH (luteinising hormone) and creatinine, permitting identification of the LH peak. Serum levels of oestradiol ($E_2$, pmol/l) and progesterone (LP $P_4$, nmol/l) were taken on the day of the LH peak (day 1) and on the day preceding the peak (day 0). 24 hours after the onset of the LH surge, the mean number of follicles greater than 16 mm in diameter was determined by a pelvic ultrasound scan, using a real-time sector scanning machine.

The results were as follows, expressed as mean values and Standard Deviations:-

| | Clomiphene citrate | Control | En isomer | Zu isomer |
|---|---|---|---|---|
| $E_2$ day 0 | 3388 ± 530 | 1022 ± 141 | 2595 ± 500 | 1176 ± 171 |
| $E_2$ day 1 | 3772 ± 614 | 1236 ± 158 | 3362 ± 638 | 1232 ± 169 |
| LP $P_4$ | 95 ± 14 | 43 ± 4 | 95 ± 15 | 44 ± 8 |
| Follicles | 2.4 ± 0.3 | 1.2 ± 0.3 | 2.1 ± 0.3 | 1.2 ± 0.2 |

It will be seen that the En isomer, at half the dosage of clomiphene citrate, gave practically the same follicle number. The Zu isomer gave no improvement over the control.

**Claims**

1) Use of (2-{para(2-chloro-1,2-diphenylvinyl) phenoxy}triethylamine or a bioequivalent derivative thereof from which the Zu isomer is substantially absent, to prepare a pharmaceutical composition for the treatment of female infertility.

2) Use according to claim 1 wherein said bioequivalent derivative thereof is an acid addition salt containing a pharmacologically acceptable anion.

3) Use according to claim 2 wherein said acid addition salt is the citrate.

4) Use according to claim 1, 2 or 3 to prepare a composition for administration to an anovulatory woman, free from any residual effects of prior treatment with clomiphene.

5) Use according to any of claims 1 to 4 to prepare a composition for administration at a dosage of from about 20 to about 50 mg per diem, calculated as the citrate.

6) Use according to any of claims 1 to 5 to prepare a composition for administration during the early follicular phase of the menstrual cycle.

7) Use according to claim 6 to prepare a composition for administration on days 3 to 7 of the menstrual cycle.

8) A method for the treatment of infertility in an anovulatory woman, which comprises administering to said woman a pharmacologically effective amount of (2-{para-(2-chloro-1,2-diphenylvinyl) phenoxy}triethylamine or a bioequivalent derivative thereof from which the Zu isomer is substantially absent.